# EUROPÄISCHE PATENTSCHRIFT

(11) **EP 2 938 250 B1**
(45) Veröffentlichungstag und Bekanntmachung des Hinweises auf die Patenterteilung: **07.06.2017**
(21) Anmeldenummer: 14719614.1
(22) Anmeldetag: 08.04.2014
(51) Int. Cl.: A61B 5/00

(54) **VORRICHTUNG UND VERFAHREN ZUR INDUKTION VON NICHTGEWEBESCHÄDIGENDEN REIZEN ZUM TESTEN EINER MECHANISCH INDUZIERTEN SCHMERZEMPFINDUNG EINES PROBANDEN**
DEVICE AND METHOD FOR INDUCING STIMULUS WHICH DOES NOT DAMAGE TISSUE FOR TESTING A MECHANICALLY INDUCED SENSATION OF PAIN IN A TEST SUBJECT
DISPOSITIF ET PROCÉDÉ POUR INDUIRE DES STIMULI N'ENTRAÎNANT PAS DE DOMMAGE TISSULAIRE, POUR TESTER UNE SENSATION DE DOULEUR INDUITE MÉCANIQUEMENT CHEZ UN SUJET D'EXPÉRIENCE

(30) Priorität: 10.04.2013 DE 102013006209
(43) Veröffentlichungstag der Anmeldung: 04.11.2015
(73) Patentinhaber: Universität Heidelberg, 69117 Heidelberg (DE)
(72) Erfinder: KROLL, Richard, 69115 Heidelberg (DE); MAGERL, Walter, 55270 Essenheim (DE); TREEDE, Rolf-Detlef, 55127 Mainz (DE)
(74) Vertreter: Müller-Boré & Partner Patentanwälte PartG mbB
(86) Internationale Anmeldenummer: PCT/EP2014/000938
(87) Internationale Veröffentlichungsnummer: WO 2014/166626

(56) Entgegenhaltungen:
- EP-A1- 1 166 717
- EP-A1- 1 249 202
- EP-A1- 2 371 274
- FR-A1- 2 967 342
- US-A1- 2005 154 329
- US-A1- 2006 015 045

## Beschreibung

Die vorliegende Erfindung betrifft eine Vorrichtung und ein Verfahren zur Induktion von nicht-gewebsschädigenden Reizen zum Testen einer mechanisch induzierten Schmerzempfindung eines Probanden.

Bei der Untersuchung von Patienten mit neuropathischen Erkrankungen, welche insbesondere Erkrankungen des peripheren Nervensystems sind, wird häufig eine Messung der Schmerzempfindlichkeit des Patienten bzw. Probanden durchgeführt. Dies erfolgt z.B. durch Aufbringen von Reizen auf die Hautoberfläche des Probanden. Weiter werden solche Messungen der Schmerzempfindlichkeit beispielsweise bei Patienten mit chronischen Rückenschmerzen, sowie bei Patienten, die an chronischer postherpetischer Neuralgie leiden, durchgeführt.

Die quantitative Messung der Schmerzempfindlichkeit eines Probanden wird in der Regel nach einem international in der Forschung anerkannten Prinzip (Teil des Quantative Sensory Testing QST) durchgeführt. Die Messung wird insbesondere mit Hilfe von standardisierten Applikatoren bzw. Stumpfen Nadeln auf Messinggewichten in Gleithülsen durchgeführt. Entsprechende Applikatoren sind z.B. in der DE 103 31 250 B3 beschrieben.

Es ist eine Aufgabe der Erfindung eine Vorrichtung und ein Verfahren zur Induktion von nicht-gewebsschädigenden Reizen zum Testen einer mechanisch induzierten Schmerzempfindung eines Probanden bereitzustellen, welche eine einfache und zuverlässige Durchführung von Tests erlauben.

Diese Aufgabe wird gemäß der Erfindung gelöst durch eine Vorrichtung mit den in Anspruch 1 angegebenen Merkmalen und ein Verfahren mit den in Anspruch 9 angegebenen Merkmalen. Bevorzugte Ausführungsformen sind Inhalt der abhängigen Ansprüche.

Gemäß der Erfindung wird eine Vorrichtung zur Induktion von nicht-gewebsschädigenden Reizen zum Testen einer mechanisch induzierten Schmerzempfindung eines Probanden bereitgestellt, welche umfasst:
eine Reiz-Induktions-Einheit zum Induzieren von nicht-gewebsschädigenden Reizen an einer zu untersuchenden Körperpartie des Probanden;
wobei die Reiz-Induktions-Einheit einen Applikator und eine Vertikal-Verstelleinrichtung zum Verändern der Position des Applikators in einer vertikalen Richtung umfasst; eine Steuer- bzw. Regeleinheit zum Steuern bzw. Regeln der Reiz-Induktions-Einheit; und
eine Positioniereinheit, an welcher zumindest die Reiz-Induktions-Einheit angeordnet ist, und welche ausgelegt ist, um an der zu untersuchenden Körperpartie des Probanden angeordnet zu werden,
wobei die Reiz-Induktions-Einheit ferner eine Horizontal-Verstelleinheit zum Verändern der Position des Applikators in einer horizontalen Richtung umfasst. Die Reiz-Induktions-Einheit ist vorzugsweise fest mit der Positioniereinheit verbunden. Mit Hilfe der Positioniereinheit kann die Reiz-Induktions-Einheit während der Durchführung eines Tests fest bzw. unverschiebbar an einer zu testenden Körperpartie des Probanden angeordnet werden.

Die Positioniereinheit hat insbesondere Kontakt zur Hautoberfläche des Probanden. Ferner ermöglicht die Positioniereinheit vorteilhafterweise eine genaue Positionierung der Reiz-Induktions-Einheit in Bezug auf die zu untersuchende Körperpartie des Probanden. Es wird insbesondere ein unverschiebliches Bezugsystem zwischen Reiz-Induktions-Einheit und der zu untersuchenden Körperpartie des Probanden bereitgestellt.

Durch festes bzw. unverschiebbares Anordnen der Reiz-Induktions-Einheit an einer Körperpartie des Probanden mit Hilfe der Positioniereinheit wird sichergestellt, daß die Reiz-Induktions-Einheit während der Dauer eines Tests korrekt in Bezug auf die zu untersuchenden Körperpartie angeordnet ist. Dadurch, daß die Positioniereinheit an der zu untersuchenden Körperpartie des Probanden angeordnet ist, wird eine Bewegung des Probanden während des Tests ermöglicht. Die erfindungsgemäße Vorrichtung ist vorzugsweise derart ausgelegt, daß sie tragbar bzw. mobil ist und die Bewegung des Probanden nicht wesentlich einschränkt.

In einer bevorzugten Ausführungsform ist die Positioniereinheit derart an der zu untersuchenden Körperpartie des Probanden anordenbar, daß bei Bewegung und/oder Beschleunigung der zu untersuchenden Körperpartie des Probanden eine relative Verschiebung zwischen der Reiz-Induktions-Einheit und der Hautoberfläche der zu untersuchenden Körperpartie des Probanden im Wesentlichen verhindert wird.

Insbesondere kann mit Hilfe der Positioniereinheit zumindest die Reiz-Induktions-Einheit derart auf bzw. an der Hautoberfläche des Probanden angeordnet bzw. befestigt werden, daß Beschleunigungen und/oder Bewegungen der zu untersuchenden Körperpartie im Wesentlichen nicht zu einer Verschiebung zwischen der Reiz-Induktions-Einheit und der Hautoberfläche führen.

Bevorzugt ist die Positioniereinheit mit der zu untersuchenden Körperpartie des Probanden fest verbindbar. Die Positioniereinheit ist somit im Wesentlichen unverrückbar an der zu untersuchenden Körperpartie des Probanden angeordnet.

Es kann vorgesehen sein, daß die Positioniereinheit die Reiz-Induktions-Einheit zumindest teilweise umgibt.

In einer bevorzugten Ausführungsform kommunizieren die Steuer- bzw. Regeleinheit und die Reiz-Induktions-Einheit drahtlos miteinander bzw. sind miteinander drahtlos bzw. kabellos verbunden. Alternative können die Steuer- bzw. Regeleinheit und die Reiz-Induktions-Einheit auch über eine Kabelverbindung miteinander kommunizieren bzw. sind über Kabel verbunden.

Des weiteren kann die Steuer- bzw. Regeleinheit räumlich getrennt von der Reiz-Induktions-Einheit angeordnet sein. Insbesondere kann vorgesehen sein, daß die Reiz-Induktions-Einheit an bzw. in der Nähe der zu testenden Körperpartie des Probanden angeordnet ist und die Steuer- bzw. Regeleinheit entfernt von dem Probanden angeordnet ist. Alternativ kann für die Steuer- bzw. Regeleinheit eine Aufnahme, z.B. in Form einer Tasche, vorgesehen sein, welche der Proband bequem mit sich tragen kann.

Alternativ kann die Steuer- bzw. Regeleinheit an der Positioniereinheit angeordnet sein.

Der Applikator kann eine Nadel mit einer stumpfen Spitze aufweisen, insbesondere PinPrick-Nadeln. Die vertikale Richtung ist insbesondere eine Richtung im Wesentlichen senkrecht zur Hautoberfläche der Probanden. Das Verändern der Position des Applikators in der vertikale Richtung umfaßt insbesondere eine Bewegung des Applikators aus einer Entfernung auf die Hautoberfläche zu oder von dieser weg. Hierbei kann zwischen einem Zustand des Kontakts des Applikators mit der Hautoberfläche und einem Zustand, in welchem sich der Applikator nicht in Kontakt mit der Hautoberfläche befindet, unterschieden werden.

Die Vertikal-Verstelleinrichtung umfaßt bevorzugt eine Einrichtung zum Beeinflussen einer mittels des Applikators auf die Hautoberfläche der Probanden wirkenden Kraft. Vorzugsweise ist die Einrichtung zum Beeinflussen einer mittels des Applikators auf die Hautoberfläche der Probanden wirkenden Kraft derart ausgelegt, daß die Geschwindigkeit mit welcher sich der Applikator auf die Hautoberfläche des Probanden zubewegt eingestellt werden kann.

Des weiteren kann vorgesehen sein, daß sich die Bewegungsenergie der Reiz-Induktions-Einheit sukzessiv bzw. allmählich entwickelt. Die Krafteinwirkung der Reiz-Induktions-Einheit auf die Hautoberfläche des Probanden kann somit beeinflußt werden.

Die horizontale Richtung ist insbesondere eine Richtung, welche im Wesentlichen parallel zur Hautoberfläche der Probanden verläuft.

Vorzugsweise umfasst die Reiz-Induktions-Einheit ferner eine Sensoreinrichtung zum Ermitteln einer mittels des Applikators auf die Hautoberfläche der Probanden wirkenden Kraft.

Die Sensoreinrichtung kann mit der Vertikal-Verstelleinrichtung rückgekoppelt sein.

Des weiteren kann die Positioniereinheit eine Auflagefläche, welche mit der Haut des Probanden in Kontakt kommt, und eine Befestigungseinrichtung zum Befestigen an der zu untersuchenden Körperpartie des Probanden umfassen. Die Auflagefläche kann starr, elastisch oder modellierbar sein. Ferner kann die Auflagefläche variable Aufsätze umfassen. Die Befestigungseinrichtung kann eine Klebefläche aufweisen, welche vorzugsweise an der Auflagefläche angeordnet ist. Alternativ oder zusätzlich kann die Befestigungseinrichtung mittels Vakuum wirken bzw. die Positioniereinheit mittels Vakuum an der Hautoberfläche des Probanden angeordnet werden. Die Befestigungseinrichtung kann alternativ oder zusätzlich einen Gurt aufweisen zum Befestigen an der Positioniereinheit an der zu untersuchenden Körperpartie des Probanden. Die kann z.B. mit Hilfe eines Klettverschlusses oder anderen geeigneten Mechanismen erfolgen.

Gemäß der Erfindung wird ferner ein Verfahren zur Induktion von nicht-gewebsschädigenden Reizen zum Testen einer mechanisch induzierten Schmerzempfindung eines Probanden, welches insbesondere mit Hilfe einer vorstehend beschriebenen Vorrichtung durchgeführt wird, bereitgestellt, wobei das Verfahren die folgenden Schritte umfaßt:
Bereitstellen einer vorstehend beschriebene Vorrichtung,
Anordnen zumindest der Reiz-Induktions-Einheit und der eine Positioniereinheit an einer zu untersuchenden Körperpartie eines Probanden; und
Induktion von nicht-gewebsschädigenden Reizen an der zu untersuchenden Körperpartie eines Probanden.

Gemäß einer bevorzugten Ausführungsform umfaßt das Verfahren ferner den Schritt des Aufnehmens von Eingaben des Probanden in Antwort auf die induzierten Reize. Das erfindungsgemäße Verfahren wird bevorzugt an Probanden oder Patienten durchgeführt, die an neuropathischen Erkrankungen, chronischen Rückenschmerzen und/oder chronischer postherpetischer Neuralgie leiden.

Die Erfindung wird im Folgenden anhand von Ausführungsbeispielen unter Bezugnahme auf die zugehörigen Zeichnungen näher erläutert. Es zeigen:
- Fig. 1: eine schematische Gesamtdarstellung einer Vorrichtung zur Induktion von nicht-gewebsschädigenden Reizen zum Testen einer mechanisch induzierten Schmerzempfindung eines Probanden gemäß einer bevorzugten Ausführungsform der Erfindung;
- Fig. 2: eine schematische Darstellung einer Reiz-Induktions-Einheit gemäß einer bevorzugten Ausführungsform der Erfindung;
- Fig. 3A, B: schematische Darstellungen der Anordnung einer Reiz-Induktions-Einheit und einer Positioniereinheit gemäß einer bevorzugten Ausführungsform der Erfindung.

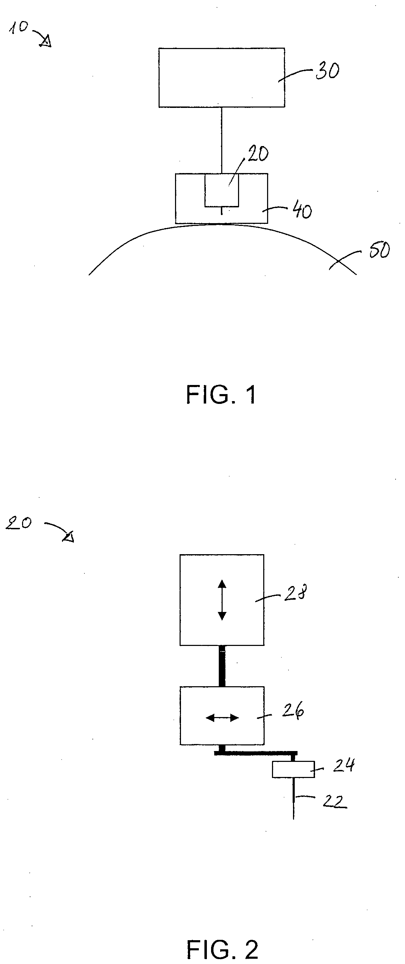

In Fig. 1 ist eine schematische Gesamtansicht einer Vorrichtung 10 zur Induktion von nicht-gewebsschädigenden Reizen zum Testen einer mechanisch induzierten Schmerzempfindung eines Probanden gemäß einer bevorzugten Ausführungsform der Erfindung gezeigt.

Die Vorrichtung 10 umfaßt eine Reiz-Induktions-Einheit 20 zum Induzieren von nicht-gewebsschädigenden Reizen an einer zu untersuchenden Körperpartie des Probanden, eine Steuer- bzw. Regeleinheit 30 zum Steuern bzw. Regeln der Reiz-Induktions-Einheit 20 und eine Positioniereinheit 40.

Fig. 2 zeigt eine schematische Ansicht der Reiz-Induktions-Einheit 20 gemäß einer bevorzugten Ausführungsform der Erfindung.

Die Reiz-Induktions-Einheit 20 gemäß Fig. 2 umfaßt einen Applikator 22, eine Sensoreinrichtung 24, eine Horizontal-Verstelleinrichtung 26 und eine Vertikal-Verstelleinrichtung 28.

Der Applikator 22 umfaßt vorzugsweise eine stumpfe Nadel, welche während eines Testvorgangs auf die Hautoberfläche eines Probanden aufgesetzt wird, um Testreize zu bewirken.

Mit Hilfe der Vertikal-Verstelleinrichtung 28 wird der Applikator 22 in der vertikalen Richtung, insbesondere einer Richtung im Wesentlichen senkrecht zur Hautoberfläche des Probanden bewegt, um Testreize zu bewirken. Die Vertikal-Verstelleinrichtung 28 ermöglicht somit eine im Wesentlichen lineare Bewegung des Applikators 22 auf die Hautoberfläche zu und von dieser weg. Die Vertikal-Verstelleinrichtung 28 ist vorzugsweise derart ausgestaltet, das die Krafteinwirkung, die der Applikator 22, insbesondere die Nadel, auf die Haut des Probanden bewirkt bzw. ausübt beeinflußt bzw. gesteuert bzw. geregelt werden kann. Beispielsweise kann die Geschwindigkeit, mit welcher sich der Applikator 22 in Richtung der Haut bewegt, beeinflußt werden. Die Vertikal-Verstelleinrichtung 28 kann z.B. einen Elektromotor umfassen, welcher die linear Bewegung des Applikators 22 bewirkt.

Alternativ kann die Vertikal-Verstelleinrichtung 28 einen Elektromagneten umfassen. In diesem Fall ist es vorteilhaft, des Weiteren eine elektronische Sanftanlauf-Einrichtung vorzusehen, um die Krafteinwirkung des Applikators 22 auf die Hautoberfläche des Probanden zu beeinflussen. Insbesondere ist es in allen Fällen von Vorteil, wenn bei Kontakt des Applikators 22 mit der Haut es zu einer sanften, nicht abrupten Krafteinwirkung auf die Haut kommt, wobei die lineare Bewegung des Applikators 22 fortgesetzt wird.

Vorzugsweise wird eine Mehrzahl von Testreizen nacheinander abgegeben. Die Zeitdauer zwischen den Testreizen kann einstellbar sein. Des Weiteren kann die Dauer eines Testreizes (Reizdauer) einstellbar bzw. veränderbar sein.

Die Horizontal-Verstelleinrichtung 26 ermöglicht eine Veränderung der Position des Applikators 22 in der horizontalen Richtung, welche insbesondere eine Richtung im Wesentlichen parallel zu der Hautoberfläche des Probanden ist. Hierbei wird die Auftreffposition der Nadel auf die Haut des Probanden verändert. Dies kann z.B. durch eine Drehbewegung der Horizontal-Verstelleinrichtung 26 erfolgen, so daß die Auftreffpositionen der Nadel im Wesentlichen ein Muster in Form eines Kreises bilden. In diesem Fall kann die Horizontal-Verstelleinrichtung 26 als Dreharm ausgebildet sein. Es kann ferner vorgesehen sein, daß der Abstand der Auftreffpositionen der Nadel variiert werden kann. Es ist ebenfalls denkbar, daß die Auftreffpositionen der Nadel ein beliebiges voreinstellbares Muster bilden. Bespiele für solche voreinstellbaren Muster sind Buchstaben, Zahlen, etc.

Es kann vorgesehen sein, daß die Horizontal-Verstelleinrichtung 26 eine Bewegung des Applikators 22 in der Ebene auf eine rotierende, lineare und/oder zweidimensionale Weise ausführen kann.

Die erfindungsgemäße Vorrichtung kann somit z.B. für Graphästhesie-Untersuchungen verwendet werden. Als Graphästhesie wird insbesondere die Fähigkeit bezeichnet, auf die Haut geschriebene Buchstaben oder Zahlen oder weiter Muster mit geschlossenen Augen zu erkennen.

Die Sensoreinrichtung 24 ist insbesondere vorgesehen, um die auf die Hautoberfläche wirkende Kraft, welche durch den Applikator 22 bewirkt wird, zu ermitteln bzw. zu messen. Vorzugsweise ist die Sensoreinrichtung 24 mit der Vertikal-Verstelleinrichtung 28 rückgekoppelt. Vorteilhafterweise ist vorgesehen, daß wenn die von der Sensoreinrichtung 24 ermittelte Kraft einen vorbestimmten und/oder voreinstellbaren Wert überschreitet, die Vertikal-Verstelleinrichtung 28 angehalten wird. Somit wird die Bewegung des Applikators 22 in Richtung der Haut des Probanden angehalten.

Des weiteren kann ein Not-Aus-Schalter (nicht gezeigt) vorgesehen sein. Dieser kann von dem Probanden betätigt werden, wenn eine unerwartete bzw. unerwünschte Krafteinwirkung auftritt.

Vorzugsweise ist die Reiz-Induktions-Einheit 20 batteriebetrieben.

Die Reihenfolge der einzelnen vorstehend beschriebenen Elemente der Reiz-Induktions-Einheit 20 kann von der in Fig. 2 gezeigten Anordnung abweichen. Der Applikator 22 sollte jedoch derart angeordnet sein, daß er mit der Haut des Probanden in Kontakt kommen kann.

Die Regel- bzw. Steuereinheit 30 umfaßt vorzugsweise einen Mikroprozessor, mittels welchem die Reiz-Induktions-Einheit 20 geregelt bzw. gesteuert wird. Insbesondere werden Signale an die Horizontal-Verstelleinrichtung 26 und Vertikal-Verstelleinrichtung 28 gesendet und von der Sensoreinrichtung 24 empfangen. Die Rückkopplung der Sensoreinrichtung 24 mit der Vertikal-Verstelleinrichtung 28 erfolgt vorzugsweise über die Regel- bzw. Steuereinheit 30.

Die Reiz-Induktions-Einheit 20 und die Regel- bzw. Steuereinheit 30 können drahtlos oder draht- bzw. kabelgebunden miteinander verbunden sein.

Die Reiz-Induktions-Einheit 20 ist an der Positioniereinheit 40 angeordnet. Vorzugsweise ist die Reiz-Induktions-Einheit 20 unverschiebbar mit der Positioniereinheit 40 verbunden. Die Positioniereinheit 40 ist ausgelegt, um an der zu untersuchenden Körperpartie des Probanden fest bzw. unverrückbar angeordnet zu werden. Insbesondere ermöglicht es die Positioniereinheit 40, die Reiz-Induktions-Einheit 20 derart in Bezug auf die zu untersuchende Hautpartie zu positionieren, daß erforderliche Testbedingungen während der gesamten Dauer des Tests eingehalten werden, auch wenn sich der Proband während des Tests bewegt. Testbedingungen sind z.B. der Abstand des Applikators 22 zur Hautoberfläche im Ruhezustand der Reiz-Induktions-Einheit 20, d.h. wenn der Applikator sich in einem zurückgezogenen Zustand befindet, in welchem er sich nicht auf die Haut zubewegt oder mit dieser Kontakt hat, oder der Auftreffwinkel des Applikators 22 auf die Hautoberfläche, welcher vorzugsweise im Wesentlichen 90° beträgt. Des Weiteren ermöglicht es die Positioniereinheit 40 die Reiz-Induktions-Einheit 20 an der Hautoberfläche des Probanden derart zu befestigen, daß Bewegungen oder Beschleunigungen der zu untersuchenden Körperpartie im Wesentlichen nicht zu einer Verschiebung zwischen der Reiz-Induktions-Einheit 20 und der Hautoberfläche führen, bzw. daß sich die Position der Reiz-Induktions-Einheit 20 in Bezug auf die Hautoberfläche im Wesentlichen nicht verändert.

Vorzugsweise ist die Positioniereinheit 40 flächig ausgestaltet, d.h. daß sie eine große Auflagefläche in Bezug auf das Gewicht aufweist und/oder die Fläche der Haut, welche von der Positioniereinheit 40 umgeben ist, groß ist. Dadurch kann weiter gewährleistet werden, daß die Positioniereinheit 40 und somit die Reiz-Induktions-Einheit 20 sicher an der zu untersuchenden Körperpartie positioniert werden kann.

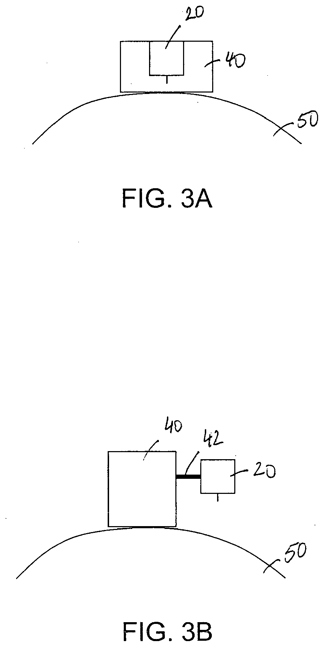

Die Positioniereinheit 40 kann jede geeignete Form aufweisen, um die Reiz-Induktions-Einheit 20 fest an der zu untersuchenden Körperpartie anzuordnen. Beispielsweise kann die Positioniereinheit 40 die Reiz-Induktions-Einheit 20 zumindest teilweise oder im Wesentlichen vollständig umgeben (Fig. 3A). In letzterem Fall sind vorzugsweise eine oder mehrere Öffnungen bzw. Aussparungen (nicht gezeigt) vorgesehen an der Seite bzw. Fläche der Positioniereinheit 40, welche mit der Haut in Kontakt kommt, die es ermöglichen, daß der Applikator 22 die Hautoberfläche des Probanden während des Tests berührt. Gemäß einer bevorzugten Ausführungsform ist die Positioniereinheit 40 in der Form eines Gehäuses um die Reiz-Induktions-Einheit 20 ausgebildet. Hierbei ist eine oder mehrere Öffnungen an der Hautkontaktseite des Gehäuses vorgesehen, um einen Kontakt des Applikators 22 mit der Hautoberfläche der Probanden zu erlauben.

Alternativ kann vorgesehen sein, daß die Positioniereinheit 40 und die Reiz-Induktions-Einheit 20 im Wesentlichen nebeneinander angeordnet sind und die Reiz-Induktions-Einheit 20 mittels einer starren Verbindungseinrichtung 42 mit der Positioniereinheit 40 verbunden ist (Fig. 3B).

Die Seite bzw. Fläche der Positioniereinheit 40, welche mit der Haut des Probanden in Kontakt kommt, im folgenden Auflagefläche genannt, kann auf verschiedene Arten ausgebildet sein. Die Auflagefläche kann starr, elastisch oder modellierbar bzw. duktil ausgebildet sein. Des weiteren kann die Auflagefläche vorzugsweise eine ergonomische Form aufweisen, um besser an dem zu untersuchenden Körperteil anzuliegen. Alternativ oder zusätzlich können variable und/oder austauschbare Aufsätze vorgesehen sein, um die Anpassung an verschiedene Körperteil und/oder Probanden zu verbessern.

Um die Positioniereinheit 40 im Wesentlichen unverschiebbar an der Hautoberfläche des Probanden anzuordnen, kann die Auflagefläche zumindest teilweise oder im Wesentlichen vollständig mit einer haftenden Schicht, z.B. einer Klebeauflage, versehen sein. Alternativ oder zusätzlich kann die Positioniereinheit 40 mittels Vakuum an der Hautoberfläche befestigt werden. Weiter alternativ oder zusätzlich kann eine Gurt oder ähnliches vorgesehen sein, um die Positioniereinheit 40 sicher an der zu testenden Körperpartie zu befestigen.

Die Steuer- bzw. Regeleinheit 30 kann räumlich getrennt von der Reiz-Induktions-Einheit 20 und der Positioniereinheit 40 vorgesehen sein. Alternativ, kann die Steuer- bzw. Regeleinheit 30 ebenfalls an der Positioniereinheit 40 angeordnet sein.

Des weiteren können die Steuer- bzw. Regeleinheit 30 und die Reiz-Induktions-Einheit 20 drahtlos oder drahtgebunden miteinander kommunizieren.

Die Vorrichtung 10 gemäß der bevorzugten Ausführung der Erfindung ist vorzugsweise derart ausgelegt, daß sie tragbar bzw. mobil ist und die Bewegung des Probanden nicht wesentlich einschränkt. Gleichzeit kann sichergestellt werden, daß die Reiz-Induktions-Einheit 20 sich sicher während der gesamten Testdauer an der gewünschten Position in Bezug auf die zu testende Körperpartie befindet.

Ein Verfahren zur Induktion von nicht-gewebsschädigenden Reizen zum Testen einer mechanisch induzierten Schmerzempfindung eines Probanden gemäß einer bevorzugten Ausführungsform der Erfindung wird vorzugsweise mit Hilfe einer vorstehend beschriebenen Vorrichtung durchgeführt. Zunächst wird zumindest ein Teil der Vorrichtung an einer zu untersuchenden Körperpartie eines Probanden angeordnet. Insbesondere wird die wie vorstehend beschrieben, die Positioniereinheit 40 und die an dieser angeordnete Reiz-Induktions-Einheit 20 an der zu untersuchenden Körperpartie unverschiebbar angeordnet. Nachfolgend werden nicht-gewebsschädigenden Reize an der zu untersuchenden Körperpartie eines Probanden induziert. Die Kraft bzw. Intensität und Position der induzierten nicht-gewebsschädigenden Reize können durch die Regel- bzw. Steuereinheit beeinflußt werden.

Des Weiteren kann vorgesehen sein, daß der Proband eine Bewertung der induzierten Reize in ein Auswert-System eingeben kann.

Die Vorrichtung und das Verfahren gemäß den bevorzugten Ausführungsformen der Erfindung ermöglichen es einheitliche und vergleichbare Testbedingungen bereitzustellen. Der Personalaufwand, welcher für entsprechende Test benötigt wird, ist gering, da lediglich eine entsprechend geschulte Person die Positioniereinheit 40, an welcher die Reiz-Induktions-Einheit 20 angeordnet ist, an der zu untersuchenden Körperpartie anbringt. Während des Testdurchlaufs ist es nicht notwendig, daß medizinisches Fachpersonal anwesend ist. Des Weiteren ist es vorteilhaft, daß sich der Proband während des Testdurchlaufs bewegen kann. Die abgegeben Testreize sind vergleichbar und wiederholbar, insbesondere kann der Test automatisiert werden. Des Weiteren ist es von Vorteil, daß der Test von der untersuchenden Person, d.h. der Person, welche mit dem Probanden den Test durchführt, unabhängig ist (Untersucherunabhängigkeit).

Es kann ferner vorgesehen sein, daß mehrere Vorrichtungen gleichzeitig an dem Probanden angeordnet bzw. fixiert werden, um simultane Tests an mehreren Hautarealen durchführen zu können.

### Bezugszeichenliste

- 10: Vorrichtung
- 20: Reiz-Induktions-Einheit
- 22: Applikator
- 24: Sensor
- 26: Horizontal-Verstelleinrichtung
- 28: Vertikal-Verstelleinrichtung
- 30: Steuer- bzw. Regeleinheit
- 40: Positioniereinheit
- 42: starre Verbindungseinrichtung
- 50: Körperpartie eines Probanden

## Patentansprüche

1. Vorrichtung (10) zur Induktion von nicht-gewebsschädigenden Reizen zum Testen einer mechanisch induzierten Schmerzempfindung eines Probanden, umfassend:
eine Reiz-Induktions-Einheit (20) zum Induzieren von nicht-gewebsschädigenden Reizen an einer zu untersuchenden Körperpartie (50) des Probanden, wobei die Reiz-Induktions-Einheit (20) einen Applikator (22) und eine Vertikal-Verstelleinrichtung (28) zum Verändern der Position des Applikators (22) in einer vertikalen Richtung umfasst;
eine Steuer- bzw. Regeleinheit (30) zum Steuern bzw. Regeln der Reiz-Induktions-Einheit (20); und
eine Positioniereinheit (40), an welcher zumindest die Reiz-Induktions-Einheit (20) angeordnet ist, und welche ausgelegt ist, um an der zu untersuchenden Körperpartie (50) des Probanden angeordnet zu werden, wobei
die Vorrichtung dadurch charakterisiert ist, dass die Reiz-Induktions-Einheit (20) ferner eine Horizontal-Verstelleinrichtung (26) zum Verändern der Position des Applikators (22) in einer horizontalen Richtung umfasst.

2. Vorrichtung (10) gemäß Anspruch 1, wobei die Positioniereinheit (40) derart an der zu untersuchenden Körperpartie (50) des Probanden anordenbar ist, daß bei Bewegung der zu untersuchenden Körperpartie (50) des Probanden eine relative Verschiebung zwischen der Reiz-Induktions-Einheit (20) und der Hautoberfläche der zu untersuchenden Körperpartie (50) des Probanden im Wesentlichen verhindert wird.

3. Vorrichtung (10) gemäß einem der vorangehenden Ansprüche, wobei die Positioniereinheit (40) mit der zu untersuchenden Körperpartie (50) des Probanden fest verbindbar ist.

4. Vorrichtung (10) gemäß einem der vorangehenden Ansprüche, wobei die Positioniereinheit (40) die Reiz-Induktions-Einheit (20) zumindest teilweise umgibt.

5. Vorrichtung (10) gemäß einem der vorangehenden Ansprüche, wobei die Steuer- bzw. Regeleinheit (30) und die Reiz-Induktions-Einheit (20) drahtlos miteinander kommunizieren/verbunden sind.

6. Vorrichtung (10) gemäß einem der vorangehenden Ansprüche, wobei der Applikator (22) eine Nadel mit einer stumpfen Spitze umfasst.

7. Vorrichtung (10) gemäß einem der vorangegangenen Ansprüche, wobei die Vertikal-Verstelleinrichtung (28) eine Einrichtung umfaßt zum Beeinflussen einer mittels des Applikators (22) auf die Hautoberfläche des Probanden wirkenden Kraft.

8. Vorrichtung (10) gemäß einem der vorangegangenen Ansprüche, wobei die Reiz-Induktions-Einheit (20) ferner umfasst:
eine Sensoreinrichtung (24) zum Ermitteln einer mittels des Applikators (22) auf die Hautoberfläche des Probanden wirkenden Kraft.

9. Verfahren zur Induktion von nicht-gewebsschädigenden Reizen zum Testen einer mechanisch induzierten Schmerzempfindung eines Probanden, welches mit Hilfe einer Vorrichtung (10) gemäß einem der vorangehenden Ansprüche durchgeführt wird, umfassend die folgenden Schritte:
Bereitstellen einer Vorrichtung (10) gemäß einem der vorangegangenen Ansprüche,
Anordnen zumindest der Reiz-Induktions-Einheit (20) und der Positioniereinheit (40) an einer zu untersuchenden Körperpartie (50) eines Probanden;
und
Induktion von nicht-gewebsschädigenden Reizen an der zu untersuchenden Körperpartie (50) eines Probanden.

10. Verfahren gemäß Anspruch 9, welches ferner umfaßt:
Aufnehmen von Eingaben des Probanden in Antwort auf die induzierten Reize.

## Claims

1. Device (10) for induction of non-tissue-damaging stimuli for testing a mechanically induced sensation of pain of a test subject, comprising:
a stimulus induction unit (20) for inducing non-tissue-damaging stimuli at a test subject's body part (50) to be examined, wherein the stimulus induction unit (20) comprises an applicator (22) and a vertical-adjustment means (28) for altering the position of the applicator (22) in a vertical direction;
a control unit (30) for controlling the stimulus induction unit (20); and
a positioning unit (40), at which at least the stimulus induction unit (20) is arranged, and which is construed so as to be arranged at the test subject's body part (50) to be examined, wherein
the device is **characterized in that** the stimulus induction unit (20) further comprises a horizontal-adjustment means (26) for altering the position of the applicator (22) in a horizontal direction.

2. The device (10) according to claim 1, wherein the positioning unit (40) is arrangeable thus at the test subject's body part (50) to be examined that in the case of movement of the test subject's body part (50) to be examined, a relative displacement between the stimulus induction unit (20) and the skin surface of the test subject's body part (50) to be examined is substantially prevented.

3. The device (10) according to any one of the preceding claims, wherein the positioning unit (40) is fixedly connectable with the test subject's body part (50) to be examined.

4. The device (10) according to any one of the preceding claims, wherein the positioning unit (40) at least partially surrounds the stimulus induction unit (20).

5. The device (10) according to any one of the preceding claims, wherein the control unit (30) and the stimulus induction unit (20) communicate with each other/are connected to one another in a wireless manner.

6. The device (10) according to any one of the preceding claims, wherein the applicator (22) comprises a needle having a blunt tip.

7. The device (10) according to any one of the preceding claims, wherein the vertical-adjustment means (28) comprises a means for influencing a force being effective on the skin surface of the test subject by means of the applicator (22).

8. The device (10) according to any one of the preceding claims, wherein the stimulus induction unit (20) further comprises:
a sensor means (24) for detecting a force being effective on the skin surface of the test subject by means of the applicator (22).

9. Method for induction of non-tissue-damaging stimuli for testing a mechanically induced sensation of pain of a test subject, which is performed by means of a device (10) according to any one of the preceding claims, comprising the following steps:
providing a device (10) according to any one of the preceding claims,
arranging at least the stimulus induction unit (20) and the positioning unit (40) at a test subject's body part (50) to be examined; and
inducing non-tissue-damaging stimuli at a test subject's body part (50) to be examined.

10. The method according to claim 9, which further comprises:
recording inputs of the test subject in response to the induced stimuli.

## Revendications

1. Dispositif (10) pour l'induction de stimuli n'endommageant pas le tissu en vue de tester une sensation de douleur induite mécaniquement d'un sujet, comprenant :
un module d'induction de stimulus (20) pour l'induction de stimuli n'endommageant pas le tissu sur une partie corporelle à examiner (50) du sujet, dans lequel le module d'induction de stimulus (20) comprend un applicateur (22) et un dispositif de réglage vertical (28) pour la modification de la position de l'applicateur (22) dans une direction verticale ;
un module de commande ou régulation (30) pour la commande ou régulation du module d'induction de stimulus (20) ; et
un module de positionnement (40) sur lequel au moins le module d'induction de stimulus (20) est disposé et qui est conçu pour être disposé sur la partie corporelle à examiner (50) du sujet, dans lequel
le dispositif est **caractérisé en ce que** le module d'induction de stimulus (20) comprend en outre un dispositif de réglage horizontal (26) pour la modification de la position de l'applicateur (22) dans une direction horizontale.

2. Dispositif (10) selon la revendication 1, dans lequel le module de positionnement (40) peut être disposé sur la partie corporelle à examiner (50) du sujet de telle sorte qu'en cas de mouvement de la partie corporelle à examiner (50) du sujet, un coulissement relatif entre le module d'induction de stimulus (20) et la surface cutanée de la partie corporelle à examiner (50) du sujet est essentiellement empêché.

3. Dispositif (10) selon une des revendications précédentes, dans lequel le module de positionnement (40) peut être connecté de manière fixe à la partie corporelle à examiner (50) du sujet.

4. Dispositif (10) selon une des revendications précédentes, dans lequel le module de positionnement (40) entoure au moins partiellement le module d'induction de stimulus (20).

5. Dispositif (10) selon une des revendications précédentes, dans lequel le module de commande ou régulation (30) et le module d'induction de stimulus (20) communiquent l'un avec l'autre/sont reliés l'un à l'autre sans fil.

6. Dispositif (10) selon une des revendications précédentes, dans lequel l'applicateur (22) comprend une aiguille avec une pointe émoussée.

7. Dispositif (10) selon une des revendications précédentes, dans lequel le dispositif de réglage vertical (28) comprend un dispositif pour l'influence d'une force agissant sur la surface cutanée du sujet au moyen de l'applicateur (22).

8. Dispositif (10) selon une des revendications précédentes, dans lequel le module d'induction de stimulus (20) comprend en outre :
un dispositif de capteur (24) pour la détermination d'une force agissant sur la surface cutanée du sujet au moyen de l'applicateur (22).

9. Procédé pour l'induction de stimuli n'endommageant pas le tissu en vue de tester une sensation de douleur induite mécaniquement d'un sujet, qui est réalisé à l'aide d'un dispositif (10) selon une des revendications précédentes, comprenant les étapes suivantes :
mise à disposition d'un dispositif (10) selon une des revendications précédentes,
agencement au moins du module d'induction de stimulus (20) et d'un des modules de positionnement (40) sur une partie corporelle à examiner (50) d'un sujet ; et
induction de stimuli n'endommageant pas le tissu sur la partie corporelle à examiner (50) d'un sujet.

10. Procédé selon la revendication 9, qui comprend en outre :
enregistrement de données du sujet en réponse aux stimuli induits.
